# EUROPEAN PATENT SPECIFICATION

(11) **EP 1 473 036 B1**
(45) Date of publication and mention of the grant of the patent: **09.08.2006**
(21) Application number: 04010435.8
(22) Date of filing: 24.04.2001
(51) Int. Cl.: A61K 31/44, C07D 471/02, A61P 43/00, C07D 471/04, C07D 235/00, C07D 221/00

(54) **Zolpidem hemitartrate solvate**
Zolpidem Hemitartrat Solvat
Zolpidem hemitartrate comme solvate

(30) Priority: 24.04.2000 US 199298 P; 22.05.2000 US 206025 P; 14.08.2000 US 225364 P
(43) Date of publication of application: 03.11.2004
(62) Divisional of application: 01930705.7
(73) Proprietor: TEVA PHARMACEUTICAL INDUSTRIES LTD., 49131 Petah Tiqva (IL)
(72) Inventor: Aronhime, Judith, Rechovot (IL); Leonov, David, Rechovot (IL); Meszaros-Sos, Erzebet, 4031 Debrecen (HU); Salyi, Szaboles, 4031 Debrecen (HU); Szabo, Csaba, 4031 Debrecen (HU); Zavurov, Shlomo, Lod (IL)
(74) Representative: Gallagher, Kirk James

(56) References cited:
- EP-A- 0 251 859
- WO-A-00/58310
- THRELFALL ET AL: "Analysis of Organic Polymorphs" ANALYST, LONDON, GB, vol. 120, October 1995 (1995-10), pages 2435-2460, XP002101807
- LEUSEN F J J: "Ab initio prediction of polymorphs" JOURNAL OF CRYSTAL GROWTH, NORTH-HOLLAND PUBLISHING CO. AMSTERDAM, NL, vol. 166, no. 1, 1 September 1996 (1996-09-01), pages 900-903, XP004053471 ISSN: 0022-0248

## Description

### FIELD OF THE INVENTION

The present invention relates to novel hydrate crystal forms of zolpidem hemitartrate and the preparation thereof.

### BACKGROUND OF THE INVENTION

Zolpidem, as a hemitartrate salt, is currently approved for the short-term treatment of insomnia in the United States under the trademark of AMBIEN. Zolpidem hemitartrate is classified as a non-benzodiazepine hypnotic of the imidazopyridine class. It has little effect on the stages of sleep in normal human subjects and is as effective as benzodiazepines in shortening sleep latency and prolonging total sleep time in patients with insomnia. The development of tolerance and physical dependence for patients using AMBIEN has been seen only very rarely and under unusual circumstances. (Goodman and Gilman's, *The Pharmacological Basis of Therapeutics* 371 (Joel G. Hardman et al., eds. 9th ed. 1996)).

Zolpidem hemitartrate (CAS Registry No. 99294-93-6) has the chemical name imidazo[1,2-a]pyridine-3-acetamide,N,N,6-trimethyl-2-(4-methylphenyl)-,(2R,3R)-2,3-di hydroxy-butanedioate and is represented by the structural formula. 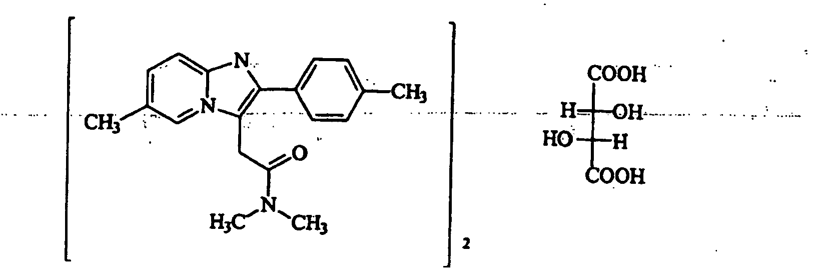 Zolpidem is among the compounds described in the following U.S. patents :4,382,938; 4,794,185; 4,356,283; 4,460,592; 4,501,745; 4,675,323; 4,808,594; and 4,847,263. The above US Patents disclose Zolpidem as having, inter alia, anxiolytic, sleep-inducing, hypnotic and anticonvulsant properties.

### SUMMARY OF THF INVENTION

In one aspect, the present invention provides for a zolpidem hemitartrate hydrate.

In one embodiment, the present invention provides a zolpidem hemitartrate monohydrate.

In another embodiment, the present invention provides a zolpidem hemitartrate dihydrate.

In another embodiment, the present invention provides a zolpidem hemitartrate trihydrate.

In another embodiment, the present invention provides a zolpidem hemitartrate tetrahydrate.

In another embodiment, the present invention provides a zolpidem hemitartrate hydrate, wherein the hydrate is a 2/3 hydrate.

In another embodiment, the present invention provides zolpidem hemitartrate monohydrate, wherein the water content is from about 2.3% to about 2.7% by weight.

In another embodiment, the present invention provides zolpidem hemitartrate dihydrate, wherein the water content is from about 5.0% to about 8.5% by weight.

In another embodiment, the present invention provides zolpidem hemitartrate (Form L) characterized by an X-ray powder diffraction pattern having peaks at about 6.8, 9.7, 17.3, 19.6 and 21.1 ± 0.2 degrees two-theta. Preferably, zolpidem hemitartrate (Form L) is further characterized by an X-ray diffraction pattern having peaks at about 7.5, 10.6, 13.2, 13.9, 16.4, 17.7, 21.6, 23.2, 23.6, 26.3, 27.1 and 29.7 ± 0.2 degrees two-theta. Preferably, the zolpidem hemitartrate is a dihydrate, preferably having a water content of about 4.3% by weight.

In one aspect, the present invention provides a use of zolpidem hemitartrate in the manufacture of medicament for the treatment of insomnia.

In another aspect, the present invention provides a process for preparing a zolpidem hemitartrate hydrate which comprises the step of granulating or slurrying a solid form of zolpidem hemitartrate in water.

In another aspect, the present invention provides a process for preparing zolpidem hemitartrate Form L, comprising the step of: (a) dissolving zolpidem hemitartrate in a solvent mixture of methanol and water; (b) precipitating zolpidem hemitartrate from the solvent mixture; and, (c) isolating zolpidem hemitartrate.

In another aspect, the present invention provides a zolpidem hemitartrate hydrate having particles up to about 200 microns in size, as measured by laser diffraction.

In one embodiment, the present invention provides a zolpidem hemitartrate hydrate having particles up to about 50 microns in size, as measured by laser diffraction.

In one embodiment, the present invention provides a pharmaceutical composition comprising a therapeutically effective amount of zolpidem hemitartrate hydrate as described above with particles up to about 200 microns in size as measured by laser diffraction and a pharmaceutically acceptable carrier.

In another embodiment, the present invention provides a pharmaceutical composition comprising a therapeutically effective amount of zolpidem hemitartrate hydrate as described above with particles up to about 50 microns in size as measured by laser diffraction and, a pharmaceutically acceptable carrier.

In one aspect, the present invention provides a pharmaceutical composition wherein the zolpidem hemitartrate hydrate is as described above.

### BRIEF DESCRIPTION OF THE FIGURES

Fig. 14. shows the X-ray diffraction pattern of zolpidem hemitartrate Form L.
Fig. 15. shows the DTG thermal profile of zolpidem hemitartrate Form L.

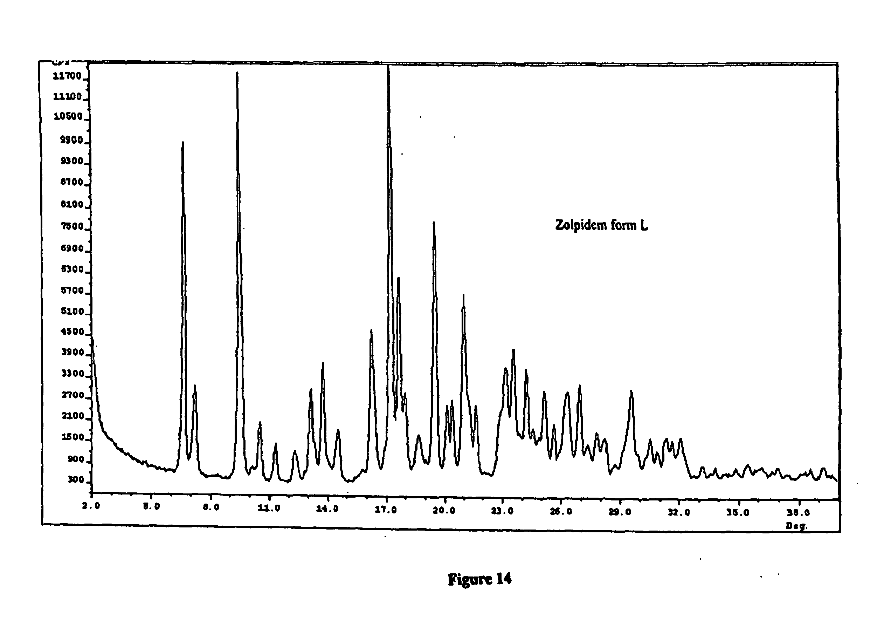

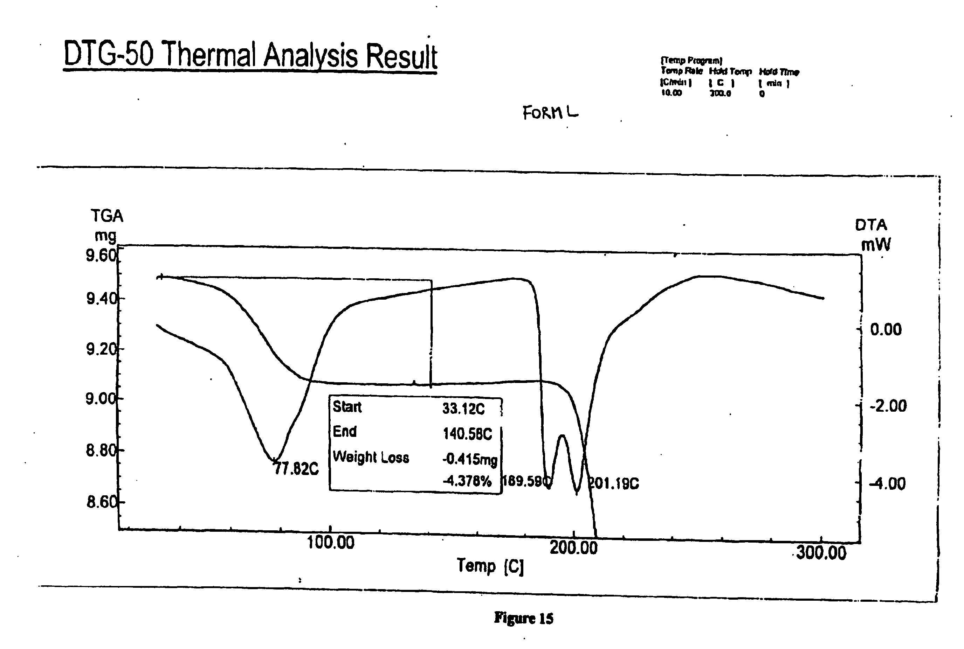

### DETAILED DESCRIPTION OF THE INVENTION

The present invention provides new hydrate crystal forms of zolpidem hemitartrate. Crystal forms of a compound can be distinguished in a laboratory by X-ray diffraction spectroscopy and by other methods such as, infrared spectrometry. It is desirable to investigate all solid state forms of a drug, including all crystal/polymorphic forms, and to determine the stability, dissolution and flow properties of each crystal/polymorphic form. For a general review of polymorphs and the pharmaceutical applications of polymorphs see G.M. Wall, *Pharm Manuf*. **3*****,*** 33 (1986); 1.K. Haleblian and W. McCrone, *J. Pharm. Sci.,* 58, 911 (1969); and J.K. Haleblian, *J. Pharm. Sci*., **64**, 1269 (1975).

As used herein, the term "zolpidem hemitartrate" includes hydrates and solvates of zolpidem hemitartrate. The term "water content" refers to the content of water based upon the Loss on Drying method (the "LOD" method) as described in Pharmacopeial Forum, Vol. 24, No. 1, p. 5438 (Jan - Feb 1998), the Karl Fisher assay for determining water content or thermogravimetric analysis (TGA). The term "equivalents of water" means molar equivalents of water. All percentages herein are by weight unless otherwise indicated. Those skilled in the art will also understand that the term "anhydrous" when used in reference to zolpidem hemitartrate describes zolpidem hemitartrate which is substantially free of water. Those skilled in the art will appreciate that the term "monohydrate" when used in reference to zolpidem hemitartrate describes a crystalline material having a water content of about 2.3% w/w. One skilled in the art will also appreciate that the term "dihydrate" when used in reference to zolpidem hemitartrate describes a crystalline material having a water content of about 4.5% w/w. One skilled in the art will also appreciate that the term "trihydrate" when used in reference to zolpidem hemitartrate describes a crystalline material having a water content of about 6.6% w/w. One skilled in the art will also appreciate that the term "tetrahydrate" when used in reference to zolpidem hemitartrate describes a crystalline material having a water content of about 8.6% w/w. One skilled in the art will also appreciate that the term "methanolate","ethanolate", "solvates of isopropranol", "solvates of butanol" or "solvates of ethylacetate" refers to Zolpidem hemitartrate in which solvent is contained within the crystal lattice in quantities above 1%. One skilled in the art will also appreciate that the term "hemiethanolate" when used in reference to zolpidem hemitartrate describes a crystalline material having an ethanol content of about 2.9% w/w.

Hydrate and solvate forms of zolpidem hemitartrate are novel and distinct from each other in terms of their characteristic powder X-ray diffraction patterns and their thermal profiles.

For the purposes of this specification, ambient temperature is from about 20°C to about 25°C.

All powder X-ray diffraction patterns were obtained by methods known in the art using a Scintag X'TRA X-ray powder diffractometer, equipped with a solid stae Si(Li) detector thermoelectrically cooled, at scanning speed of 3 ° min.⁻¹ scanning range 2-40 degrees two-theta. Copper radiation of λ = 1.5418 Å was used.

Measurement of thermal analysis are conducted for the purpose of evaluating the physical and chemical changes that may take place in a heated sample. Thermal reactions can be endothermic (eg, melting, boiling, sublimation, vaporization, desolvation, solid-solid phase transitions, chemical degradation, etc.) or exothermic (eg, crystallization, oxidative decomposition, etc.) in nature. Such methodology has gained widespread use in the pharmaceutical industry in characterization of polymorphism. The quantitative applications of thermal applications of thermal analysis have proven to be useful in characterization of polymorphic systems. The most commonly applied techniques are thermogravimetry (TGA), differential thermal analysis (DTA), and differential scanning calorimetry (DSC).

The DTA and TGA curves presented herein were obtained by methods known in the art using a DTG Shimadzu model DTG-50 (combined TGA and DTA). The weight of the samples was about 9 to about 13 mg. The samples were scanned up to about 300°C or above at a rate of 10°C/min. Samples were purged with nitrogen gas at a flow rate of 20 ml/min. Standard alumina crucibles covered lids with one hole.

Thermogravimetry analysis (TGA) is a measure of the thermally induced weight loss of a material as a function of the applied temperature. TGA is restricted to transitions that involve either a gain or a loss of mass, and it is most commonly used to study desolvation processes and compound decomposition.

Karl Fisher analysis, which is well known in the art, is also used to determine the quantity of water in a sample.

As used herein a slurry refers to a suspension of insoluble particles or slightly soluble particles in an aqueous or organic (non-aqueous) liquid, without complete dissolution of the solid.

### SYNTHESIS OF ZOLPIDEM-HEMITARTRATE

The present invention provides a methods of synthesizing zolpidem hemitartrate. Zolpidem hemitartrate base may be synthesized as disclosed in U.S. Patent No. 4,382,938. Therein, is disclosed that zolpidem base may be formed from zolpidic acid by reacting the acid with dimethyl amine, in the presence of carbonyldiimidazole. This method has several disadvantages such as low yield and formation of impurities which are difficult to remove. US Patent No. 4,382,938 also mentions the possibility of reacting zolpidic acid chloride with dimethyl amine. However no procedure for this preparation of zolpidiem is mentioned. The present patent present a procedure for the preparation of Zolpidem from zolpidic acid which has the following advantages:
- High reaction yields
- Improved purity profile of the prepared Zolpidem
- Preparation of Zolpidem from zolpidic acid in a "one pot" procedure.
Alternatively the base may be formed by reacting the zolpidic acid chloride with dimethyl amine. U.S. Patent No. 4,794,185 discloses alternative methods for synthesizing zolpidem base.

Once zolpidem base is formed, the zolpidem hemitartrate is prepared by dissolving the base in methanol and adding L(+)-tartric acid dissolved in methanol. The hemitartrate is then crystalized from methanol.

### Formation of the Acid Chloride

Preferably, formation of zolpidem base from the acid comprises a two-step reaction. In the first step, zolpidic acid chloride (II) is formed from zolpidic acid (I).

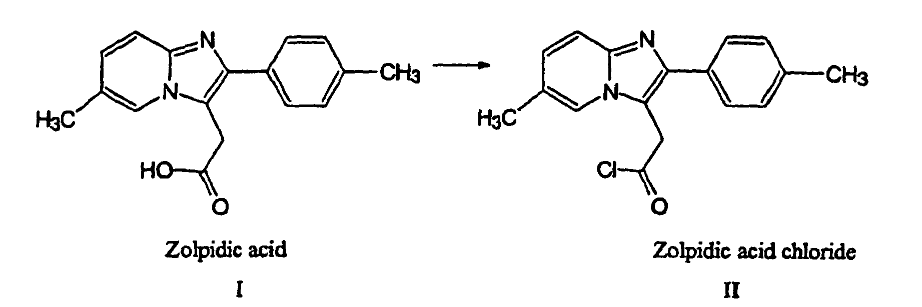

The chlorination reaction can be performed using SOCl₂, PCL₅ and POCL₃. The most preferred chlorination agent is SOCl₂ because its excess can be removed smoothly after the reaction end by distillation from the reaction mass.

Preferred solvents are aliphatic or aromatic hydrocarbons, chlorinated solvents and aprotic polar solvents and mixtures thereof. The most preferred reaction solvent is toluene containing traces of dimethylformamide (DMF). The use of toluene as reaction solvent has the following advantages:
1. The intermediate zolpidic acid chloride (II) and the final zolpidem can be isolated at whish from this solvent in such a way that the procedure can be designed as a one step or two step process.
2. In the presence of toluene the excess of SOCl₂ is easy to remove as an azeotrop SOCl₂- toluene.
3. Zolpidic acid chloride (II) precipitate from the reaction mixture during the chlorination reaction. An overchlorination of the zolpidic acid is thus avoided.

DMF can be regarded as a phase transfer catalyst of the chlorination reaction by facilitating the access of SOCl₂ to the Zolgidic acid. Also in the precipitation of Zolpidem the presence of DMF contribute to a better purification effect of the reaction solvent.

The preferred temperature range for forming the acid chloride is from about 15 to about 28° C. Most preferably the temperature a in the range of about 18 to about 22 °C.

After formation of the acid chloride, the thionyl chloride is distilled from the reaction mixture.

The preferred temperature of distillation is in the range of about 30 to about 40 °C. Most preferably the temperature of distillation is in the range of about 35 to about 40 °C.

The pressure of vacuum distillation is in the range of about 30 to about 100 mm Hg. Most preferably the pressure is in the range of about 30 to about 50 mm Hg.

### Formation of Zolpidem Base

In the second step, zolpidic acid chloride (II) is used to form zolpidem base (the compound of formula III) in a reaction with dimethyl amine as shown.

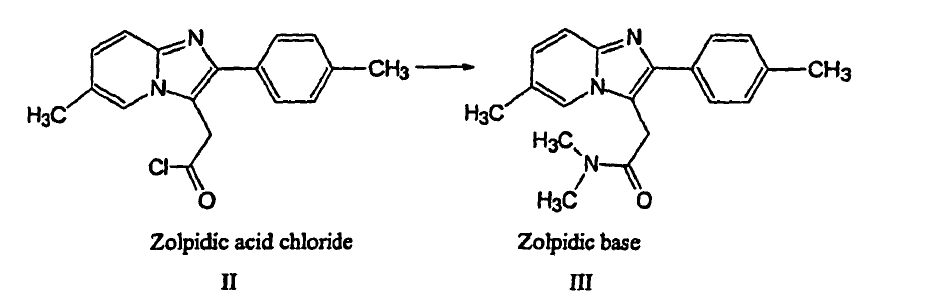

Preferred solvents are aliphatic or aromatic hydrocarbons, chlorinated solvents and aprotic polar solvents and mixtures thereof. The most preferred solvent is toluene. The use of toluene as reaction solvent has the following advantages:
1. Zolpidic acid and coloured impurities formed during the chlorination reaction are effectively removed.
2. The crystallization process in toluene is optimal.
3. Essentially pure Zolpidem (98% area% by HPLC) is obtained

Dimethylamine is preferably introduced as a gas until the pH is from about 8.5 and about 9.5.

The preferred temperature range for forming the base is from about -5 to about +3 °C. Most preferably the temperature is in the range of about -5 to about 0 °C.

After dimethylamine gas is introduced, the resulting zolpidem base forms a precipitate. After the solution is mixed for 1 hour, the solution is cooled to about -10 to about -12 °C. Typically the precipitate is then collected by filtration. However, the precipitate may be collected by any means known in the art.

The zolpidem base is then dried and used to form the hemitartrate by dissolving the base in methanol and adding L(+)-tartric acid dissolved in methanol. The hemitartrate is then crystalized from methanol.

### NOVEL HYDRATE FORMS OF ZOLPIDEM HEMITARTRATE

The present invention provides novel crystal forms of zolpidem hemitartrate which will be designated as Form L. These forms can be distinguished from the prior art form of zolpidem hemitartrate and from each other by characteristic powder X-ray diffraction patterns and thermal profiles.

The different crystal forms may also be characterized by their respective solvation state. The most commonly encountered solvates among pharmaceuticals are those of 1:1 stoichiometry. Occasionally mixed solvate species are encountered. When water or solvent is incorporated into the crystal lattice of a compound in stoichiometric proportions, the molecular adduct or adducts formed are referred to as hydrates or solvates.

### Zolpidem hemitartrate Form L

Zolpidem hemitartrate Form L ("Form L") is a dihydrate crystal form of zolpidem hemitartrate.

Zolpidem hemitartrate Form L is characterized by an X-ray diffraction pattern with peaks at about 6.8, 7.5, 9.7, 10.6, 13.2, 13.9, 16.4, 17.3, 17.7, 19.6, 21.1, 21.6, 23.2, 23.6, 26.3, 27.1 and 29.7 ± 0.2 degrees two-theta. The most characteristic peaks of Form L are at about 6.8, 9.7, 17.3, 19.6 and 21.1 ±0.2 degrees two-theta. The diffraction pattern is reproduced in Fig. 14.

The DTG thermal profile of Form L is shown in Fig. 15. The DSC thermal profile of Form L contains an endotherm of desorption at about 78 °C. The DSC thermal profile further shows a double endotherm of melting and decomposition at 190 °C and 201 °C.

The hydration state of Form L is unequivocably by TGA and Karl Fisher analysis. Form L has a weight loss of about 4.4 % mainly at 80°C which corresponds to about 2 water molecules per molecule of zolpidem hemitartrate. Karl Fischer analysis of Form L reveals about 4.3 % water. Thus, the TGA and Karl Fisher analysis indicate that Form L is a dihydrate of zolpidem hemitartrate.

### Procedures for Crystallizing Polymorphs of Zolpidem Hemitartrate

### General Description

The novel forms of zolpidem hemitartrate disclosed herein are optionally formed by: (1) exposing various solid forms of zolpidem hemitartrate to water vapor or solvent vapors; (2) suspending the crystals as a slurry of zolpidem hemitartrate particles in a solvent; (3) granulation; (4) crystallization. It will be understood by those of skill in the art that other methods may also be used to form the polymorphs disclosed herein.

### Formation of Polymorphs by Vapor Exposure

Optionally, vapor treatment may be performed by placing, about 0.1 g to about 0.2 g of a solid form of zolpidem hemitartrate in a small open container. The container can be a flat 5 cm (or less) diameter dish. The container can be optionally a bottle of a volume of about 10 ml. The open bottle is optionally introduced into a chamber of a volume from about 50 ml to about 150 ml. The chamber may be a bottle. The chamber preferably contains about 5 to about 30 ml of a solvent. The chamber is sealed creating a solvent saturated atmosphere. Preferably, the sample is then stored for a time period ranging from about 5 to about 10 days. Most preferably the sample is stored for about 7 days. When the solvent is water the degree of chamber humidity may be regulated using salts or salt solutions such as potassium sulphate, zinc nitrate, potassium acetate, ammonium sulphate, and the chamber is a 20x20x10 cm size sealed chamber apposite for this purpose (hygroscopicity chamber). The solid zolpidem hemitartrate is then analyzed.

### Formation of Polymorphs by Slurry

Forming the suspension optionally includes mixing solid zolpidem hemitartrate with a solvent in which complete dissolution does not occur. The mixture is optionally stirred for a period of time needed to achieve the desired transformation, and the solid compound collected and analyzed.

### Formation of Polymorphs by Granulation

Granulation optionally includes mixing solid zolpidem hemitartrate with a minimal amount of solvent insufficient to dissolve the material, and stirring the mixture at room temperature for the time needed to cause the desired transformation. The mixture is optionally stirred for a period of time and the compound collected and analyzed.

### SMALL PARTICLES OF ZOLPIDEM HEMITARTRATE

The present invention also provides zolpidem hemitartrate having a relatively small particle size and a corresponding relatively large surface area.

It has long been recognized that when a pharmaceutical composition containing a drug which is orally administered to subjects, a dissolution step is essential for the drug to be absorbed through gastrointestinal tract. A drug may have insufficient bioavailability because of the poor solubility in the gastrointestinal tract, consequently the drug passes through the site of absorption before it completely dissolves in the fluids.

Bioavailability, particularly of slightly soluble active compounds is highly dependent on the surface area of the particles and the surface area is inversely related to the size of the compound. Thus particles having relatively small particle size have a relatively greater surface area and an increased solubility rate in gastrointestinal tract.

Small zolpidem hemitartrate particles can be achieved using methods well known in the art. (See US Patents Nos. 4,151,273; 4,196,188; 4,302,446; 4,332, 721; 4,840,799; and 5,271,944) Micronization as provided in Example 33 is one method of generating small zolpidem hemitartrate particles. Particle size was measured by a laser diffraction instrument (Malvern Mastersizer S). The sample was analysed after proper dispersion in a solution of dioctylsulfosuccinate sodium salt in hexane (0.02% w/w).

In one embodiment, the invention provides zolpidem hemitartrate in which substantially all zolpidem hemitartrate particles have a particle size of up to about 200 micrometer. It will be understood by those of skill in the art that this embodiment includes pharmaceutical compositions containing a therapeutically effective amount of zolpidem hemitartrate.

According to another embodiment, the present invention provides zolpidem hemitartrate particles in which substantially all zolpidem hemitartrate particles, have a particle size of up to about 50 microns. It will be understood by those of skill in the art that this embodiment includes pharmaceutical compositions containing a therapeutically effective amount of zolpidem hemitartrate.

### A PHARMACEUTICAL COMPOSITION CONTAINING ZOLPIDEM HEMITARTRATE

According to another aspect, the present invention relates to a pharmaceutical composition comprising one or more of the novel crystal forms of zolpidem hemitartrate disclosed herein and at least one pharmaceutically acceptable excipient. Such pharmaceutical compositions may be administered to a mammalian patient in a dosage form.

The dosage forms may contain one or more of the novel forms of zolpidem hemitartrate or, alternatively, may contain one or more of the novel forms of zolpidem hemitartrate as part of a composition. Whether administered in pure form or in a composition, the zolpidem hemitartrate form(s) may be in the form of a powder, granules, aggregates or any other solid form. The compositions of the present invention include compositions for tableting. Tableting compositions may have few or many components depending upon the tableting method used, the release rate desired and other factors. For example, compositions of the present invention may contain diluents such as cellulose-derived materials like powdered cellulose, microcrystalline cellulose, microfine cellulose, methyl cellulose, ethyl cellulose, hydroxyethyl cellulose, hydroxypropyl cellulose, hydroxypropylmethyl cellulose, carboxymethyl cellulose salts and other substituted and unsubstituted celluloses; starch; pregelatinized starch; inorganic diluents such calcium carbonate and calcium diphosphate and other diluents known to one of ordinary skill in the art. Yet other suitable diluents include waxes, sugars (*e*.*g*. lactose) and sugar alcohols like mannitol and sorbitol, acrylate polymers and copolymers, as well as pectin, dextrin and gelatin.

Other excipients contemplated by the present invention include binders, such as acacia gum, pregelatinized starch, sodium alginate, glucose and other binders used in wet and dry granulation and direct compression tableting processes; disintegrants such as sodium starch glycolate, crospovidone, low-substituted hydroxypropyl cellulose and others; lubricants like magnesium and calcium stearate and sodium stearyl fumarate; flavorings; sweeteners; preservatives; pharmaceutically acceptable dyes and glidants such as silicon dioxide.

Dosage forms may be adapted for administration to the patient by oral, buccal, parenteral, ophthalmic, rectal and transdermal routes. Oral dosage forms include tablets, pills, capsules, troches, sachets, suspensions, powders, lozenges, elixirs and the like. The novel forms ofzolpidem hemitartrate disclosed herein also may be administered as suppositories, ophthalmic ointments and suspensions, and parenteral suspensions, which are administered by other routes. The most preferred route of administration of the zolpidem hemitartrate forms of the present invention is oral.

Capsule dosages will contain the solid composition within a capsule which may be coated with gelatin. Tablets and powders may also be coated with an enteric coating. The enteric-coated powder forms may have coatings comprising phthalic acid cellulose acetate, hydroxypropylmethyl cellulose phthalate, polyvinyl alcohol phthalate, carboxymethylethylcellulose, a copolymer of styrene and maleic acid, a copolymer of methacrylic acid and methyl methacrylate, and like materials, and if desired, they may be employed with suitable plasticizers and/or extending agents. A coated tablet may have a coating on the surface of the tablet or may be a tablet comprising a powder or granules with an enteric-coating.

The currently marketed form of zolpidem hemitartrate (AMBIEN) are a 5 and a 10 mg tablet which includes the following inactive ingredients: hydroxypropyl methylcellulose, lactose, magnesium stearate, microcrystalline cellulose, polyethylene glycol, sodium starch glycolate, titanium dioxide; the 5 mg tablet also contains FD&C Red No. 40, iron oxide colorant, and polysorbate 80.

The function and advantage of these and other embodiments of the present invention will be more fully understood from the examples below. The following examples are intended to illustrate the benefits of the present invention, but do not exemplify the full scope of the invention.

### EXAMPLES

### EXAMPLE 1:

### Synthesis of zolpidem base.

5 g (17.7 mmol) of Zolpidic acid is suspended in 50 ml of toluene and 0.15 ml of dimethylformamide. This mixture is cooled to 15-28 °C. 1.7 ml (23.3 mmol) of thionyl chloride is added into the mixture at this temperature for 1 h, then it is stirred for 4 hrs at 35-40 °C.

After formation of acid chloride the thionyl chloride excess is removed by distillation. The volume of the reaction mixture is adjusted to 50 ml by toluene, then it is cooled to -5-0 °C, then dimethylamine gas is introduced into the reaction mixture until the pH is 8.5-9.5. Precipitation of Zolpidem base starts almost immediately.

The suspension is cooled to -10-(-12) °C and mixed for 1 h. The crude product is filtered and washed consecutively with toluene, 5 % cooled water solution of NH4CO3and cooled water. The product is dried under vacuum 4.1 g (assay 97.6 % by HPLC, yield 80 %) Zolpidem base is obtained.

### EXAMPLE 2

### Synthesis of zolpidem base.

5 g (17.7 mmol) of Zolpidic acid is suspended in 50 ml of toluene and 0.3 ml of dried dimethylformamide. This mixture is cooled to 15-28 °C. 1.4 ml (19.5 mmol) of thionyl chloride is added into the mixture at this temperature for 1 h, then it is stirred for 4 hrs at 20-25 °C.

After formation of acid chloride the thionyl chloride excess is removed by distillation.
The volume of the reaction mixture is adjusted to 50 ml by toluene, then it is cooled to -5-0 °C, then dimethylamine gas is introduced into the reaction mixture until the pH is 8.5-9.5. Precipitation of Zolpidem base starts ahnost immediately.

The suspension is cooled to 0-5 °C and mixed for 1 h. The crude product is filtered and washed consecutively with toluene, 5 % cooled water solution of NH4CO3 and cooled water. The product is dried under vacuum .4.4 g (assay 94.6 % by HPLC, yield 70.7 %) Zolpidem base is obtained.

### EXAMPLE 32

### Formation of zolpidem hemitartrate Form L by Crystallization.

Zolpidem hemitartrate (5 g) was dissolved in a mixture of 43.6 ml of methanol and 3.4 ml water (methanol:water ratio is 13:1) at 60°C. The solution was filtered and cooled to room temperature. Upon reaching 30°C precipitation of Zolpidem hemitartrate started. The suspension was mixed at room temperature for 3 hrs then methanol was evaporated by vacuum distillation. The suspension was stored for 12 hrs at 0-5°C. The sample was filtered and dried in vacuum (150 mbar) at 40°C for 16 hrs. The XRD analysis showed the product to be a novel zolpidem hemitartrate designated Form L.

### EXAMPLE 33

### Micronization of zolpidem hemitartrate

Pure dry zolpidem hemitartrate was micronized in an air jet micronizer (CHRISPRO Jetmill MC-200KX, BD). The feeding rate was set at 9.0 kg/hr. The feeding air pressure was set at 6.0 bar. The grinding air pressure was set 3.5 bar. The particle size of the micronized zolpidem hemitartrate was found to be less than 20 microns Malvern laser diffraction Mastersizer S.

## Claims

1. Zolpidem hemitartrate hydrate.

2. The zolpidem hemitartrate hydrate of claim 1, wherein the hydrate is a monohydrate.

3. The zolpidem hemitartrate hydrate of claim 1, wherein the hydrate is a dihydrate.

4. The zolpidem hemitartrate hydrate of claim 1, wherein the hydrate is a trihydrate.

5. The zolpidem hemitartrate hydrate of claim 1, wherein the hydrate is a tetrahydrate.

6. The zolpidem hemitartrate hydrate of claim 1, wherein the hydrate is a 2/3 hydrate.

7. The zolpidem hemitartrate hydrate of claim 2, wherein the zolpidem hemitartrate has a water content from about 2.3 % to about 2.7 % by weight

8. The zolpidem hemitartrate hydrate of claims 3 to 5, wherein the zolpidem hemitartrate has a water content from about 5.0% to about 8.5% by weight.

9. A process for preparing zolpidem hemitartrate hydrate according to any preceding claim which comprises the step of granulating or slurrying a solid form of zolpidem hemitartrate in water.

10. The zolpidem hemitartrate hydrate of claim 1 or 3, wherein the zolpidem hemitartrate is **characterized by** an X-ray pattern diffraction having peaks at 6.8, 9.7, 17.3, 19.6 and 21.1 ± 0.2 degrees two-theta.

11. The zolpidem hemitartrate of claim 10, further **characterized by** an X-ray pattern diffraction having peaks at 7.5, 10.6, 13.2, 13.9, 16.4, 17.7, 21.6, 23.2, 23.6, 26.3, 27.1 and 29.7 ± 10.2 degrees two-theta.

12. The zolpidem hemitartrate hydrate of claims 10 or 11, wherein the water content is about 4.3% by weight.

13. A process for preparing zolpidem hemitartrate according to claims 10 to 12, comprising the step of:
(a) dissolving zolpidem hemitartrate in a solvent mixture of methanol and water;
(b) precipitating zolpidem hemitartrate from the solvent mixture; and,
(c) isolating zolpidem hemitartrate.

14. The process of claim 13, wherein the solvent mixture of methanol and water is at a ratio of about 13 parts methanol to about 1 part water.

15. The zolpidem hemitartrate hydrate of claims 1 to 8 and 10 to 12 having particles up to about 200 microns in size as measured by laser diffraction.

16. Zolpidem hemitartrate of claim 15 having particles up to about 50 microns in size.

17. A pharmaceutical composition comprising a therapeutically effective amount of zolpidem hemitartrate according to any of claims 1 to 8, 10 to 12, 15 and 16 and a pharmaceutically acceptable carrier.

18. Use of a zolpidem hemitartrate according to any of claims 1 to 8,10 to 12 and 15 to 17, in the manufacture of a medicament for treating insomnia.

## Patentansprüche

1. Zolpidem-Hemitartrat-Hydrat.

2. Zolpidem-Hemitartrat-Hydrat nach Anspruch 1, wobei das Hydrat ein Monohydrat ist.

3. Zolpidem-Hemitartrat-Hydrat nach Anspruch 1, wobei das Hydrat ein Dihydrat ist.

4. Zolpidem-Hemitartrat-Hydrat nach Anspruch 1, wobei das Hydrat ein Trihydrat ist.

5. Zolpidem-Hemitartrat-Hydrat nach Anspruch 1, wobei das Hydrat ein Tetrahydrat ist.

6. Zolpidem-Hemitartrat-Hydrat nach Anspruch 1, wobei das Hydrat ein 2/3-Hydrat ist.

7. Zolpidem-Hemitartrat-Hydrat nach Anspruch 2, wobei das Zolpidem-Hemitartrat einen Wassergehalt von etwa 2,3 Gew.-% bis etwa 2,7 Gew.-% hat.

8. Zolpidem-Hemitartrat-Hydrat nach einem der Ansprüche 3 bis 5, wobei das Zolpidem-Hemitartrat einen Wassergehalt von etwa 5,0 Gew.-% bis etwa 8,5 Gew.-% hat.

9. Verfahren zur Herstellung von Zolpidem-Hemitartrat-Hydrat nach einem der vorangegangenen Ansprüche, welches die Stufe umfaßt, bei der man eine feste Form von Zolpidem-Hemitartrat granuliert oder in Wasser aufschlämmt.

10. Zolpidem-Hemitartrat-Hydrat nach einem der Ansprüche 1 oder 3, wobei das Zolpidem-Hemitartrat durch ein Pulverröntgenbeugungsmuster mit Peaks bei 6,8, 9,7, 17,3, 19,6 und 21,1 ± 0,2 Grad Zwei-Theta **gekennzeichnet** ist.

11. Zolpidem-Hemitartrat nach Anspruch 10, weiterhin **gekennzeichnet durch** ein Pulverröntgenbeugungsmuster mit Peaks bei 7,5, 10,6, 13,2, 13,9, 16,4, 17,7, 21,6, 23,2, 23,6, 26,3, 27,1 und 29,7 ± 0,2 Grad Zwei-Theta.

12. Zolpidem-Hemitartrat-Hydrat nach einem der Ansprüche 10 oder 11, wobei der Wassergehalt etwa 4,3 Gew.-% beträgt.

13. Verfahren zur Herstellung von Zolpidem-Hemitartrat nach einem der Ansprüche 10 bis 12, welches die folgenden Stufen umfaßt:
(a) Lösen von Zolpidem-Hemitartrat in einem Lösungsmittelgemisch aus Methanol und Wasser,
(b) Präzipitieren von Zolpidem-Hemitartrat aus dem Lösungsmittelgemisch und
(c) Isolieren von Zolpidem-Hemitartrat.

14. Verfahren nach Anspruch 13, wobei das Lösungsmittelgemisch aus Methanol und Wasser ein Verhältnis von etwa 13 Teilen Methanol zu etwa 1 Teil Wasser hat.

15. Zolpidem-Hemitartrat-Hydrat nach einem der Ansprüche 1 bis 8 und 10 bis 12 mit Teilchen von bis zu etwa 200 Mikrometern Größe, gemessen durch Laserdiffraktion.

16. Zolpidem-Hemitartrat nach Anspruch 15 mit Teilchen von bis zu etwa 50 Mikrometern Größe.

17. Pharmazeutische Zusammensetzung, die eine therapeutisch wirksame Menge an Zolpidem-Hemitartrat nach einem der Ansprüche 1 bis 8, 10 bis 12, 15 und 16 und einen pharmazeutisch verträglichen Träger umfaßt.

18. Verwendung eines Zolpidem-Hemitartrats nach einem der Ansprüche 1 bis 8, 10 bis 12 und 15 bis 17 bei der Herstellung eines Medikaments zur Behandlung von Insomnie.

## Revendications

1. Hémitartrate de zolpidem hydraté.

2. Hémitartrate de zolpidem hydraté selon la revendication 1, dans lequel l'hydrate est un monohydrate.

3. Hémitartrate de zolpidem hydraté selon la revendication 1, dans lequel l'hydrate est un dihydrate.

4. Hémitartrate de zolpidem hydraté selon la revendication 1, dans lequel l'hydrate est un trihydrate.

5. Hémitartrate de zolpidem hydraté selon la revendication 1, dans lequel l'hydrate est un tétrahydrate.

6. Hémitartrate de zolpidem hydraté selon la revendication 1, dans lequel l'hydrate est un 2/3 d'hydrate.

7. Hémitartrate de zolpidem hydraté selon la revendication 2, dans lequel l'hémitartrate de zolpidem a une teneur en eau d'environ 2,3% à environ 2,7% en poids.

8. Hémitartrate de zolpidem hydraté selon les revendications 3 à 5, dans lequel l'hémitartrate de zolpidem a une teneur en eau d'environ 5,0% à environ 8,5% en poids.

9. Procédé de préparation d'hémitartrate de zolpidem hydraté selon l'une quelconque des revendications précédentes, qui comprend l'étape de granulation ou de mise sous forme de pâte d'une forme solide d'hémitartrate de zolpidem dans de l'eau.

10. Hémitartrate de zolpidem hydraté selon la revendication 1 ou 3, dans lequel l'hémitartrate de zolpidem est **caractérisé par** un diagramme de diffraction des rayons X ayant des pics à un angle deux thêta de 6,8, 9,7, 17,3, 19,6 et 21,1 ± 0,2 degrés.

11. Hémitartrate de zolpidem selon la revendication 10, **caractérisé de plus par** un diagramme de diffraction des rayons X ayant des pics à un angle deux thêta de 7,5, 10,6, 13,2, 13,9, 16,4, 17,7, 21,6, 23,2, 23,6, 26,3, 27,1 et 29,7 ± 0,2 degrés.

12. Hémitartrate de zolpidem hydraté selon les revendications 10 ou 11, dans lequel la teneur en eau est d'environ 4,3% en poids.

13. Procédé de préparation d'hémitartrate de zolpidem selon les revendications 10 à 12, comprenant les étapes de :
(a) dissolution de l'hémitartrate de zolpidem dans un mélange de solvants de méthanol et d'eau ;
(b) précipitation de l'hémitartrate de zolpidem à partir du mélange de solvants ; et
(c) isolement de l'hémitartrate de zolpidem.

14. Procédé selon la revendication 13, dans lequel le mélange de solvants de méthanol et d'eau est présent selon un rapport d'environ 13 parties de méthanol pour environ 1 partie d'eau

15. Hémitartrate de zolpidem hydraté selon les revendications 1 à 8 et 10 à 12 ayant des particules d'une taille allant jusqu'à environ 200 µm, telle que mesurée par diffraction laser.

16. Hémitartrate de zolpidem selon la revendication 15 ayant des particules d'une taille allant jusqu'à environ 50 µm.

17. Composition pharmaceutique comprenant une quantité efficace du point de vue thérapeutique d'hémitartrate de zolpidem selon l'une quelconque des revendications 1 à 8, 10 à 12, 15 et 16 et un véhicule acceptable du point de vue pharmaceutique.

18. Utilisation d'un hémitartrate de zolpidem selon l'une quelconque des revendications 1 à 8, 10 à 12 et 15 à 17, dans la fabrication d'un médicament destiné à traiter l'insomnie.
